(19) Europäisches Patentamt
European Patent Office
Office européen des brevets

(11) Veröffentlichungsnummer : **0 332 969 B1**

(12) # EUROPÄISCHE PATENTSCHRIFT

(45) Veröffentlichungstag der Patentschrift : **09.10.91 Patentblatt 91/41**

(51) Int. Cl.$^5$ : **C07C 53/00, C07C 51/54**

(21) Anmeldenummer : **89103873.9**

(22) Anmeldetag : **06.03.89**

(54) **Verfahren zur Herstellung von Monocarbonsäureanhydriden.**

(30) Priorität : **17.03.88 DE 3808867**

(43) Veröffentlichungstag der Anmeldung : **20.09.89 Patentblatt 89/38**

(45) Bekanntmachung des Hinweises auf die Patenterteilung : **09.10.91 Patentblatt 91/41**

(84) Benannte Vertragsstaaten : **AT BE CH DE FR GB IT LI NL**

(56) Entgegenhaltungen :
**EP-A- 0 180 799**
**EP-A- 0 180 802**
**EP-A- 0 203 286**

(73) Patentinhaber : **HOECHST AKTIENGESELLSCHAFT**
**Postfach 80 03 20**
**W-6230 Frankfurt am Main 80 (DE)**

(72) Erfinder : **Luft, Gerhard, Prof. Dr.**
**Ludwigstrasse 141a**
**W-6109 Mühltal (DE)**
Erfinder : **Trabold, Peter, Dr.**
**Ahornweg 19a**
**W-6110 Dieburg (DE)**

EP 0 332 969 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Trägerkatalysators bei Temperaturen von 130 bis 400°C und Drücken von 1-150 bar, wobei im Trägerkatalysator eine Organosiliciumverbindung mit Alkoxy- oder Halogengruppen sowie mit Organostickstoff-, Organophosphor-, Organoarsen-, Organoschwefel- oder Mercaptogruppen als mehrfunktioneller Haftvermittler einerseits an ein Trägermaterial und andererseits an eine Edelmetallverbindung aus der Gruppe VIII des Periodensystems gebunden ist.

Ein derartiges, in der Gasphase mit einem Trägerkatalysator arbeitendes Verfahren ist bereits aus der DE-Offenlegungsschrift 3440647 A1 bekannt, welche die bei den Flüssigphaseverfahren auftretenden Nachteile, z.B. die schwierige Abtrennung und Rückführung von suspendiertem und teilweise gelöstem Katalysator und ggf. Promotor, vermeidet.

Aus der DE-OS 3511050 A1 ist außerdem ein sehr ähnliches Verfahren bekannt, bei dem allerdings im Trägerkatalysator das Trägermaterial mit der Lösung einer Edelmetallchelatverbindung, die aus der Edelmetallverbindung und einem Chelator mit Organostickstoff-, Organophosphor-, Organoarsen-, Organoschwefel- oder Mercaptogruppen gebildet ist, lediglich imprägniert wurde.

Es ist Aufgabe der vorliegenden Erfindung, den Chelator so zu modifizieren, daß er als mehrfunktioneller Haftvermittler wirkt und sich Standzeit (Aktivitätsdauer) und Selektivität des Trägerkatalysators bei gleichem Trägermaterial deutlich verbessern.

Im einzelnen ist das Verfahren der Erfindung dadurch gekennzeichnet, daß eine chelatbildende Organosiliciumverbindung der allgemeinen Formel

a)

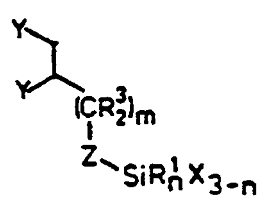

oder b)

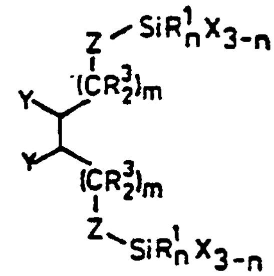

als mehrfunktioneller Haftvermittler eingesetzt wird, wobei

X = —Cl, —Br oder —$OR^2$;
Y = —$NR^4_2$, ein stickstoffhaltiger Arylrest, —$PR^4_2$, $AsR^4_2$, —$SR^4$ oder —SH ;
Z = null, Arylen, Phenylen (ggf. ortho-, meta- oder parasubstituiert)
$R^1$ = $C_1$ bis $C_5$-Alkyl ;
$R^2$ = $C_1$ bis $C_5$-Alkyl oder —$C_6H_5$ ;
$R^3$ = —H, $C_1$ bis $C_3$-Alkyl ;
$R^4$ = $C_1$ bis $C_6$-Alkyl, $C_5$ oder $C_6$-Cycloalkyl oder —$C_6H_5$ oder —$CH_2C_6H_5$, die ggf. mit Halogen-, Methoxy-, Ethoxy- oder $C_1$ bis $C_3$-Alkylgruppen substituiert sind ;
n = 0 oder 1 oder 2 ;
m = 2 bis 6, vorzugsweise 2 bis 4.

Darüberhinaus kann das Verfahren der Erfindung wahlweise und bevorzugt dadurch gekennzeichnet sein, daß

a) im Trägerkatalysator die chelatbildende Organosiliciumverbindung als mehrfunktioneller Haftvermittler einerseits an das Trägermaterial und andererseits abwechselnd an die Edelmetallverbindung und an eine Nichtedelmetallverbindung aus der 6. oder 8. Nebengruppe des Periodensystems der Elemente gebunden ist ;

b) der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält ;
c) der Trägerkatalysator ein anorganisches oxidisches Trägermaterial oder einen Aktivkohleträger enthält, deren restliche aktive Hydroxygruppen durch Veresterung oder Veretherung desaktiviert wurden ;
d) der Trägerkatalysator zusammen 0,01 bis 50 Gew%, vorzugsweise 0,1 bis 20 Gew%, Edelmetallverbindung, Haftvermittler und ggf. Nichtedelmetallverbindung enthält ;
e) der Trägerkatalysator in einer Korngröße von 1 bis 20 mm eingesetzt wird.

Als Katalysatorträger kommen bevorzugt anorganische Oxide wie z.B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle in Frage, welche BET-Oberflächen von 1-1000 $m^2/g$, vorzugsweise 30-400 $m^2/g$, haben und stets noch aktive OH-Gruppen aufweisen müssen. Diese OH-Gruppen reagieren mit der oder den funktionellen Gruppen X des Haftvermittlers unter Bildung von Sauerstoffbrücken zwischen Träger und Haftvermittler.

Ebenso wie gemäß DE-OS 3511050 und 3440647 sind die Promotoren der 5. oder 6. Hauptgruppe in den erfindungsgemäß eingesetzten Haftvermittlern chemisch gebunden. Sie bilden selbst eine funktionelle Gruppe, die die Edelmetallverbindungen aus der Gruppe VIII und ggf. Nichtedelmetallverbindungen aus der 6. oder 8. Nebengruppe chelatisieren.

Es ist ein Vorteil, daß die zur Steigerung der Aktivität und der Selektivität der Trägerkatalysatoren notwendigen Promotoren aus der 5. oder 6. Hauptgruppe des Periodensystems der Elemente eine funktionelle Gruppe Y in den mehrfunktionellen Haftvermittlern bilden und somit bis zur maximalen Konzentration, die durch die Anzahl der OH-Gruppen auf der Trägeroberfläche bestimmt ist, fixiert werden können. Deshalb entfällt eine Abtrennung und Rückführung dieser z.B. Organostickstoff- oder Organophosphorpromotoren.

Das Verfahren der Erfindung zur Herstellung von Monocarbonsäureanhydriden weist gegenüber den eingangs beschriebenen bekannten Verfahren höhere Selektivitäten und besonders bei Langzeitbelastung höhere Standzeiten des Trägerkatalysators auf.

Ein weiterer Vorteil des Verfahrens der Erfindung ist darin zu sehen, daß es die Möglichkeit bietet, Edelmetallchelate chemisch auf Trägeroberflächen zu fixieren. Weiterhin zeigen die auf dem Trägermaterial aufgetragenen modifizierten Edelmetall-Chelatverbindungen und ggf. Nichtedelmetall-Chelatverbindungen noch höhere Schmelzpunkte (240-270°C) als die in den DE-OS 3440647 und 3511050 beschriebenen Komplexe, was zu einer höheren thermischen Stabilität der Katalysatoren bzw. zu einer Erhöhung des Anwendungsbereichs von 20 bis 50°C führt.

Das Verfahren der Erfindung dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1-4 C-Atomen darstellt.

In den allgemeinen Formeln für die Organosiliciumverbindungen, welche als Haftvermittler (Spacer) in Frage kommen, bedeutet X vorzugsweise —$OR^2$ und insbesondere Methoxy oder Ethoxy. Sofern n nicht null ist, bedeutet $R^1$ einen unverzweigten Alkylrest, insbesondere Methyl, Ethyl oder Propyl.

Die Trägermaterialien wurden bereits genannt ; als Mischoxide kommen z.B. $Cr_2O_3$-$Al_2O_3$, $WO_3$-$Al_2O_3$, MgO-$Al_2O_3$, $SiO_2$-$Al_2O_3$ oder $ZrO_2$-$Al_2O_3$ in Frage. Der Trägerkatalysator enthält bevorzugt 0.05 bis 5 Gew% Edelmetall.

Als Edelmetallverbindungen können bei der Herstellung des Trägerkatalysators z.B. folgende Verbindungen eingesetzt werden :

Rhodium : $RhCl_3$, $RhCl_3 \cdot 3\,H_2O$, $RhBr_3$, $RhI_3$, $Rh(NO_3)_3$, $Rh_2(CO)_4Cl_2$, $Rh_2(CO)_4Br_2$, $Rh(CO)_4I_2$, $[P(C_6H_5)_3]_3RhCl$, $[P(C_6H_5)_3]_2Rh(CO)Cl$, $Rh_6(CO)_{16}$, $Rh_4(CO)_{12}$, $Rh_2(O_2CCH_3)_4$, $[RhCl(C_8H_{12})]_2$ ;

Iridium : $IrCl_3$, $[Ir(CO)_3Cl]_2$, $Ir[P(C_6H_5)_3]_2(CO)Cl$, $Ir_4(CO)_{12}$, $[IrCl(C_8H_{12})]_2$, $Cl(CO)_2Irpyr$ (pyr = $C_6H_5N$) ;

Palladium : $PdCl_2$, $PdBr_2$, $PdI_2$, $(CH_3CO_2)_2Pd[P(C_6H_5)_3]_2$, $PdCl_2[P(C_6H_5)_3]_2$, $Pd(O_2CCH_3)_2$, $PdCl_2(C_8H_{12})$, $(C_6H_5CN)_2PdCl_2$ ;

Ruthenium : $RuCl_3$, $Ru_3(CO)_{12}$, $RuCl_2[P(C_6H_5)_3]_3$, $RuCl_2(CO)_2[P(C_6H_5)_3]_2$, $[RuCl_2(CO)_3]_2$.

Als Nichtedelmetallverbindungen aus der 6. oder 8. Nebengruppe, insbesondere Cr, Ni, aber auch W, Fe, Co, die ebenfalls mit den Chelatoren reagieren, kommen z.B. ferner in Frage :

Chrom : $Cr(CO)_6$, $CrCl_3$, $C_7H_8Cr(CO)_3$.

Nickel : $Ni(CO)_4$, $[P(C_6H_5)_3]_2Ni(CO)_2$, $NiCl_2$, $Ni(C_8H_{12})_2$.

Als Nichtedelmetallverbindungen aus der 1. bis 3. Hauptgruppe oder der 4. bis 6. oder der 8. Nebengruppe des Periodensystems, vorzugsweise des Li, Na, Mg, Ca, Al, Ti, Zr, V, Cr, W, Fe, Co, Ni, können z.B. Hydroxide, Carbonate, Carbonyle, Hydride, Halogenide und andere Salze eingesetzt werden. Diese Verbindungen unedler Metalle können zusätzlich z.B. als Lösung durch Imprägnierung, auf den Katalysatorträger aufgebracht werden.

Zur Herstellung des erfindungsgemäß eingesetzten Trägerkatalysators muß zuerst der mehrfunktionelle Haftvermittler, d.h. die chelatbildende Organosiliciumverbindung, mit den funktionellen Gruppen Y bereitgestellt werden. Dieser kann nach oder in Analogie zu Literaturangaben dargestellt werden. Im allgemeinen wird dann eine der genannten Edelmetallverbindungen aus der Gruppe VIII und ggf. eine der genannten Nichtedelmetallverbindungen aus der 6. oder 8. Nebengruppe in Lösung mit dem Haftvermittler in Verbindung gebracht, wobei Chelatverbindungen entstehen, die durch ihre Organosiliciumfunktion zur chemischen Fixierung geeignet sind.

Anschließend erfolgt die reaktive Anlagerung des edelmetallhaltigen Chelats an die OH-Gruppen des Trägermaterials unter Austritt einer Gruppe X als XH (z.B. HCl, HBr oder $HOR^2$). Dies wird durch 24- bis 100 stündiges Erhitzen auf Rückflußtemperatur der in einem unpolaren Lösemittel suspendierten Komponenten erreicht.

Alle weiteren Einzelheiten zu Synthesen ergeben sich aus der Beschreibung der Katalysatorherstellung.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 mol, vorzugsweise 1 bis 100 mol, je 1 mol Jod(verbindung) oder Brom(verbindung). Die Temperatur der Reaktionszone wird so gewählt, daß das Reaktionsgemisch bei jedem beliebigen Umsatz gasförmig vorliegt. Bevorzugt wird die Temperatur zwischen 150 und 250°C gewählt. Der bevorzugte Druck liegt zwischen 5 und 30 bar.

Die Verweilzeit des Reaktionsgemisches am festen Trägerkatalysator beträgt 1 bis 1000 s, bevorzugt 1 bis 180 s. Die Umsetzung kann in einem senkrecht angeordneten und mit Trägerkatalysator gefüllten Strömungsrohr oder auch in einem Rühr- oder Schüttelautoklaven erfolgen, der den Trägerkatalysator enthält. Man führt die Carbonylierung im allgemeinen unter wasserfreien Bedingungen durch, doch sind geringe Wassermengen, wie sie in den handelsüblichen Ausgangsstoffen vorkommen, zulässig, sollten aber 1 mol%, berechnet auf die Ausgangsstoffe, nicht übersteigen. Auch wird die Carbonylierung durch geringe Mengen Methanol in den Ausgangsstoffen nicht beeinträchtigt. Ebensowenig stört Wasserstoff, der in kleinen Mengen im handelsüblichen Kohlenmonoxid vorhanden sein kann.

Der aus der Carbonylierungszone abströmende Reaktionsgemisch ist gasförmig und enthält Kohlenmonoxid, Methyljodid, Essigsäurenahydrid, nichtumgesetztes Methylacetat oder Dimethylether und ggf. sehr geringe Mengen Essigsäure.

Das gasförmige Reaktionsgemisch wird abgekühlt, Essigsäureanhydrid und ggf. Essigsäure auskondensiert und die nicht kondensierten Stoffe wie CO, Methyljodid, Methylacetat oder Dimethylether in die Reaktionszone zurückgeführt. Die umgesetzten Anteile von Ester oder Ether sowie CO werden fortlaufend ersetzt.

Die einfache Abtrennung der Anhydride durch Kühlung des abströmenden Reaktionsgemisches und Rückführung der nicht kondensierbaren Gase stellt, wie bei den genannten, bekannten Verfahren, einen wesentlichen Vorteil dar, da dies ohne komplizierte Trennoperationen erfolgen kann. Der Trägerkatalysator wird nicht verunreinigt und verbleibt in der Reaktionszone, was den gesamten Verfahrensablauf bedeutend vereinfacht.

Beispiele

Rührautoklavversuche :

Man verwendet einen 0,25 Liter fassenden Rührautoklaven aus korrosionsfreiem Edelstahl (Hastelloy C) mit den erforderlichen Zu- und Ableitungen, der einen drehbaren Katalysatorkorb enthält.

Die Carbonsäureester oder Dialkylether werden gasförmig in Gegenwart des bewegten, festen Trägerkatalysators mit CO-Gas umgesetzt. Der Trägerkatalysator befindet sich in dem drehbaren Katalysatorkorb, der gleichzeitig für die Durchmischung der Gase sorgt.

Der Autoklav wird mit 2,5 ml eines flüssigen Gemisches aus 20 Volumenteilen Methyljodid und 80 Volumenteilen Ester oder Ether beschickt und auf Reaktionstemperatur erhitzt. Die Carbonylierung wird durch Aufpressen von Kohlenmonoxid eingeleitet. Der CO-Druck wird durch regelmäßiges Nachdrücken konstant gehalten.

Die Einzelheiten der Versuchsdurchführungen ergeben sich aus den Beispielen.

Beispiel 1

2 ml (1,86 g) Essigsäuremethylester, 0,5 ml (1,14 g) Methyljodid und 7,2 g Katalysator Nr. 1 werden im Autoklaven mit Kohlenmonoxid bei 15 bar CO-Druck und 180°C umgesetzt. Nach 1 h Reaktionszeit ergibt sich eine Katalysatorleistung von 19,7 g $Ac_2O/g_{Rh} \times h$, bei einer Selektivität von 95%.

Beispiel 2

2 ml (1,86 g) Essigsäuremethylester, 0,5 ml (1,14 g) Methyljodid und 7,9 g Katalysator Nr. 2 werden im Autoklaven mit Kohlenmonoxid bei 15 bar CO-Druck und 180°C umgesetzt. Nach 1 h Reaktionszeit ergibt sich eine Katalysatorleistung von 18,8 g $Ac_2O/g_{Rh} \times h$, bei einer Selektivität von 96%.

Beispiel 3

2 ml (1,86 g) Essigsäuremethylester, 0,5 ml (1,14 g) Methyljodid und 6,5 g Katalysator Nr. 3 werden im Autoklaven mit Kohlenmonoxid bei 15 bar CO-Druck und 180°C umgesetzt. Nach 1 h Reaktionszeit ergibt sich eine Katalysatorleistung von 41,0 g $Ac_2O/g_{Rh} \times h$, bei einer Selektivität von 89%.

Strömungsrohrversuch :

Beispiel 4

Ein Stahlrohr von 20 mm Durchmesser und 400 mm Länge wird als Strömungsrohr senkrecht angeordnet und mit 50,6 g Katalysator Nr. 1 gefüllt. Bei einem Druck von 12 bar und einer Temperatur von 180°C werden stündlich 8 Nl CO (Nl = Liter, gemessen bei 1,013 bar und 0°C), sowie ein verdampftes Gemisch (12,8 ml Flüssigkeit) aus Methyljodid und Methylacetat (Molverhältnis 1 : 4) durch das Strömungsrohr geleitet.

Das abströmende Reaktionsgemisch wird gaschromatographisch on-line analysiert. Hierbei ergibt sich eine Raum-Zeit-Ausbeute von 12,5 g $Ac_2O/g_{Rh} \times h$, bei einer Selektivität von 97%.

Unter diesen Reaktionsbedingungen wurde die Carbonylierung 280 Stunden durchgeführt, wobei der eingesetzte Trägerkatalysator keinen Aktivitätsverlust zeigte.

Beschreibung der Katalysatorherstellung

In allen Fällen wurden die Katalysatorträger zur Aktivierung zuvor bei 200°C und 0,1 mbar 10 h getrocknet. Nach Aufbringen der Metallkomponente erhitzte man die Katalysatoren 8 h mit Chlortrimethylsilan zum Sieden und trocknete anschließend bei 0,1 mbar und 100°C. Sämtliche Synthesen wurden in Argonatmosphäre unter Ausschluß von Luftsauerstoff und Wasser ausgeführt. Alle verwendeten Lösemittel wurden zuvor über Molekularsieb 4 A oder wenn möglich mit Benzophenonnatrium getrocknet.

Das in den nachfolgenden Formeln gebrauchte Symbol "ϕ" steht für den Phenylrest ($C_6H_5$).

Katalysator Nr. 1

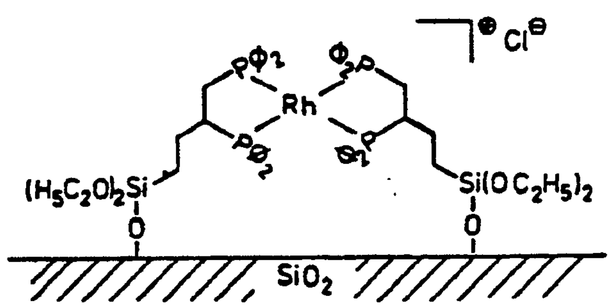

62,9 g aktivierte Siliciumdioxidpellets 1/8" × 1/8" (95% $SiO_2$) mit einer inneren Oberfläche nach BET von 68 $m^2/g$ und einem Porenvolumen von 0,43 ml/g wurden mit 150 ml einer Lösung aus 722 mg des Komplexes <u>4</u> in Toluol versetzt. Die gelbe Suspension wurde 24 h auf Rückflußtemperatur erhitzt, wobei sich das Lösemittel

vollständig entfärbte. Nach Abziehen des Toluols unter reduziertem Druck wurde der Katalysator bei 0,1 mbar und 150°C 6 h getrocknet und anschließend 24 h in einer Soxhlet-Apparatur mit Benzol extrahiert. Nach der Extraktion konnte im Benzol kein Rhodium nachgewiesen werden.
Charakterisierung : hellgelbe Pellets
Rh-Gehalt : 0,09 Gew.%

Syntheseweg des Rhodiumkomplexes 4

1,2-Dichlor-4-(triethoxysilyl)butan (2) :

0,1 mol 1-Butenylmagnesiumbromid (1) in 100 ml Tetrahydrofuran versetzt man tropfenweise mit 0,5 mol Tetraethoxysilan und erhitzt 5 h auf Rückflußtemperatur. Anschließend wird die erhaltene Suspension filtriert und das Lösemittel abgezogen. Den Rückstand nimmt man in Dichlormethan auf und leitet bei 0°C so lange Chlor ein bis sich die Lösung schwach gelb färbt. Durch Abziehen des Lösemittels und anschließende Vakuumdestillation erhält man 2 in 64%iger Ausbeute.

1,2-Bis(diphenylphosphino)-4-(triethoxysilyl)-butan (3) :

3 wird durch Umsetzung doppeltmolarer Menge Natriumdiphenylphosphid in Dioxan mit 2, gelöst in Tetrahydrofuran, bei Raumtemperatur synthetisiert [analog 1,2-Bis(diphenylphosphino)ethan ; siehe K. Issleib und D.-W. Müller, Chem. Ber. 92 , 3175 (1959)]. Ausbeute 72%.

[1,2-Bis(diphenylphosphino)-4-(triethoxysilyl)-butan]rhodium(I)-chlorid (4) :

4 mmol 3, gelöst in Benzol, werden unter Rühren zu einer Lösung von 1 mmol Dichlorotetracarbonyldirhodium in Benzol getropft. Abziehen des Lösungsmittels und Umkristallisieren aus Hexan liefert analysenreinen Komplex 4. Ausbeute 94%. Vgl. Synthese von [1,2-Bis(diphenylphosphino)-ethan]rhodium(I)-chlorid ; A. Sacco et al., J. Chem. Soc. (London), 3274 (1964).

Katalysator Nr. 2

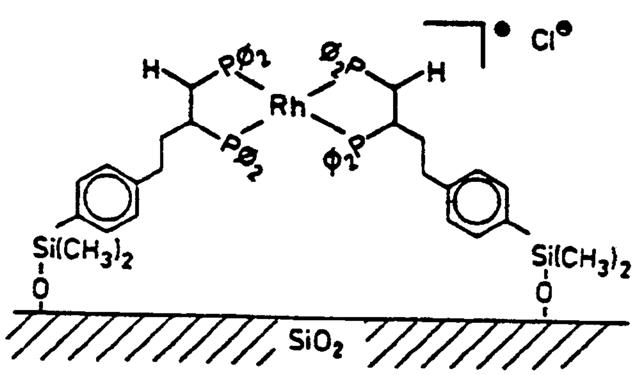

12,7 g aktivierte Siliciumdioxidpellets 1/8" x 1/8" (95% $SiO_2$) mit einer inneren Oberfläche nach BET von 68 $m^2/g$ und einem Porenvolumen von 0,43 ml/g wurden mit 50 ml einer Lösung aus 133 mg des Komplexes 9 in Toluol versetzt. Die gelbe Suspension wurde 24 h auf Rückflußtemperatur erhitzt, wobei sich das Lösemittel vollständig entfärbte. Nach Abziehen des Toluols unter reduziertem Druck wurde der Katalysator bei 0,1 mbar und 150°C 6 h getrocknet und anschließend 24 h in einer Soxhlet-Apparatur mit Benzol extrahiert. Nach der Extraktion konnte im Benzol kein Rhodium nachgewiesen werden.
Charakterisierung : hellgelbe Pellets
Rh-Gehalt : 0,08 Gew.%

Syntheseweg des Rhodiumkomplexes 9

1,2-Dichlor-4-(4-chlorphenyl)butan (6) :

6 kann durch Umsetzung von 4-(4-Chlorphenyl)buten (5) mit Chlor bei 0°C in Dichlormethan synthetisiert

werden. Ausbeute 93%.

1,2-Bis(diphenylphosphino)-4-(4-chlorphenyl)-butan (7) :

7 wird durch Umsetzung doppeltmolarer Menge Natriumdiphenylphosphid in Dioxan mit 6, gelöst in Tetrahydrofuran, bei Raumtemperatur mit 82% Ausbeute synthetisiert [analog 1,2-Bis(diphenylphosphino)ethan ; siehe K. Issleib und D.-W. Müller, Chem. Ber. 92, 3175 (1959)].

1,2-Bis(diphenylphosphino)-4-[4-dimethylethoxysilyl)-phenyl]butan (8) :

0,05 mol 7 werden in Tetrahydrofuran zur Arylmagnesiumchloridverbindung umgesetzt ; siehe R. D. Rieke u. S. E. Bales, J. Am. Chem. Soc. 96, 1775 (1974) ; J. P. Collmann et al., J. Am. Chem. Soc. 105, 7288 (1983). Anschließend tropft man unter Rühren und Eiskühlung 0,25 mol Diethoxydimethylsilan zu, läßt auf Raumtemperatur erwärmen und erhitzt schließlich 5 h auf Rückflußtemperatur. Die Reaktionsmischung wird filtriert ; Lösemittel und überschüssiges Diethoxydimethylsilan werden im Vakuum abgezogen. Den öligen Rückstand kristallisiert man aus Hexan und erhält 8 in 68%iger Ausbeute.

[1,2-Bis(diphenylphosphino)-4-[4-dimethylethoxysilyl)phenyl]butan]rhodium(I)-chlorid (9) :

4 mmol 8, gelöst in Benzol, werden unter Rühren zu einer Lösung von 1 mmol Dichlorotetracarbonyldirhodium in Benzol getropft. Abziehen des Lösungsmittels und Umkristallisieren aus Hexan liefert analysenreinen Komplex 9. Ausbeute 95%. Vgl. Synthese von [1,2-Bis(diphenylphosphino)-ethan]-rhodium(I)-chlorid ; A. Sacco et al., J. Chem. Soc. (London), 3274 (1964).

Katalysator Nr. 3

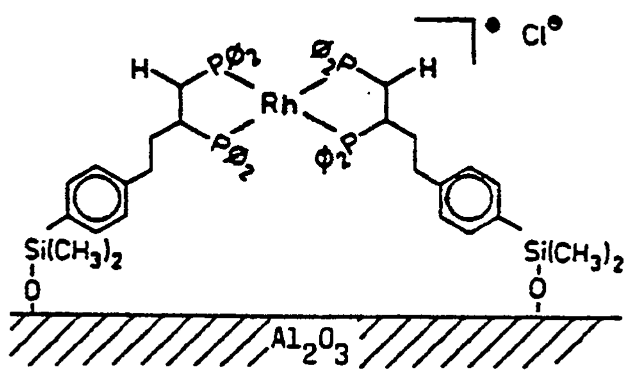

11.7 g aktivierte Aluminiumoxidkugeln (99% $Al_2O_3$) mit einem Durchmesser von 3 mm, einer inneren Oberfläche nach BET von 125 $m^2/g$ und einem Porenvolumen von 0,9 ml/g wurden mit 50 ml einer Lösung aus 156 mg des Komplexes 9 in Toluol versetzt. Die gelbe Suspension wurde 24 h auf Rückflußtemperatur erhitzt, wobei sich das Lösemittel vollständig entfärbte. Nach Abziehen des Toluols unter reduziertem Druck wurde der Katalysator bei 0,1 mbar und 150°C 6 h getrocknet und anschließend 24 h in einer Soxhlet-Apparatur mit Benzol extrahiert. Nach der Extraktion konnte im Benzol kein Rhodium nachgewiesen werden.
Charakterisierung : hellgelbe Kugeln
Rh-Gehalt : 0,1 Gew.%

## Patentansprüche

1. Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1 bis 4 C-Atomen bedeutet, mit Kohlenmonoxid in der Gasphase in Gegenwart von Jod oder Brom oder deren Verbindungen als Reaktionspromotor sowie in Gegenwart eines Trägerkatalysators bei Temperaturen von 130 bis 400°C und Drücken von 1 bis 150 bar, wobei im Trägerkatalysator eine Organosiliciumverbindung mit Alkoxy- oder Halogengruppen sowie mit Organostickstoff-, Organophosphor-, Organoarsen- Organoschwefel- oder Mercaptogruppen als mehrfunktioneller Haftvermittler einerseits an ein

Trägermaterial und andererseits an eine Edelmetallverbindung aus der Gruppe VIII des Periodensystems gebunden ist, dadurch gekennzeichnet, daß eine chelatbildende Organosiliciumverbindung der allgemeinen Formel

a)

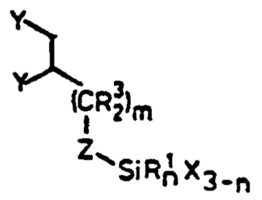

oder b)

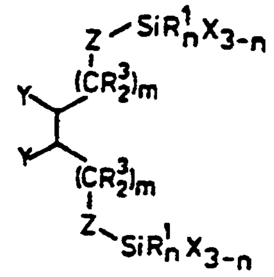

als mehrfunktioneller Haftvermittler eingesetzt wird, wobei

X = $-Cl$, $-Br$ oder $-OR^2$;
Y = $-NR_2^4$, ein stickstoffhaltiger Arylrest, $-PR_2^4$, $AsR_2^4$, $-SR^4$ oder $-SH$;
Z = null, Arylen, Phenylen (ggf. ortho-, meta- oder parasubstituiert)
$R^1$ = $C_1$ bis $C_5$-Alkyl;
$R^2$ = $C_1$ bis $C_5$-Alkyl oder $-C_6H_5$;
$R^3$ = $-H$, $C_1$ bis $C_3$-Alkyl;
$R^4$ = $C_1$ bis $C_6$-Alkyl, $C_5$ oder $C_6$-Cycloalkyl oder $-C_6H_5$ oder $-CH_2C_6H_5$, die ggf. mit Halogen-, Methoxy-, Ethoxy- oder $C_1$ bis $C_3$-Alkylgruppen substituiert sind;
n = 0 oder 1 oder 2;
m = 2 bis 6, vorzugsweise 2 bis 4.

2. Verfahren nach Anspruch 1, dadurch gekennzeichnet, daß im Trägerkatalysator die chelatbildende Organosiliciumverbindung als mehrfunktioneller Haftvermittler einerseits an das Trägermaterial und andererseits abwechselnd an die Edelmetallverbindung und an eine Nichtedelmetallverbindung aus der 6. oder 8. Nebengruppe des Periodensystems der Elemente gebunden ist.

3. Verfahren nach Anspruch 1 oder 2, dadurch gekennzeichnet, daß der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält.

4. Verfahren nach einem der Ansprüche 1-3, dadurch gekennzeichnet, daß der Trägerkatalysator ein anorganisches oxidisches Trägermaterial oder einen Aktivkohleträger enthält, deren restliche aktive Hydroxygruppen durch Veresterung oder Veretherung desaktiviert wurden.

5. Verfahren nach einem der Ansprüche 1-4, dadurch gekennzeichnet, daß der Trägerkatalysator zusammen 0,01 bis 50 Gew%, vorzugsweise 0,1 bis 20 Gew%, Edelmetallverbindung, Haftvermittler und ggf. Nichtedelmetallverbindung enthält.

6. Verfahren nach einem der Anspruche 1-5, dadurch gekennzeichnet, daß der Trägerkatalysator in einer Korngröße von 1 bis 20 mm eingesetzt wird.

## Claims

1. A process for the preparation of a monocarboxylic anhydride of the formula $(RCO)_2O$ by reacting a carboxylic acid ester or dialkyl ether of the formula RCOOR or ROR, where R in each case denotes the same alkyl radical having 1 to 4 carbon atoms, with carbon monoxide in the gas phase in the presence of iodine or bromine or a compound thereof as reaction promoter, and in the presence of a supported catalyst, at a temperature of from 130 to 400°C and a pressure of from 1 to 150 bar, where, in the supported catalyst, an organosilicon compound containing alkoxy or halogen groups and containing organonitrogen, organophosphorus, organoarsenic, organosulfur or mercapto groups, is bonded, as a polyfunctional coupling agent on the one hand to a support

material and on the other hand to a noble-metal compound from group VIII of the Periodic Table, which comprises employing, as the polyfunctional coupling agent, a chelate-forming organo-silicon compound of the formula

a) 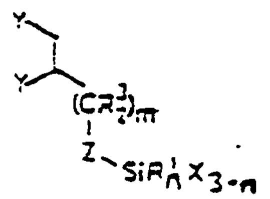 or b) 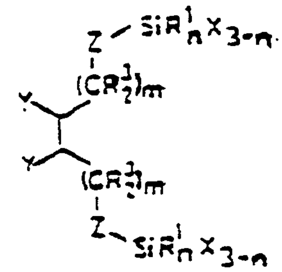

where

X = —Cl, —Br or —OR$^2$ ;
Y = —NR$^4_2$, a nitrogen-containing aryl radical, —PR$^4_2$, AsR$^4_2$, —SR$^4$ or —SH ;
Z = zero, arylene or phenylene (which may be ortho-, meta- or para-substituted) ;
R$^1$ = $C_1$ to $C_5$-alkyl ;
R$^2$ = $C_1$ to $C_5$-alkyl or —$C_6H_5$ ;
R$^3$ = —H or $C_1$ to $C_3$-alkyl ;
R$^4$ = $C_1$ to $C_8$-alkyl, $C_5$ or $C_6$-cycloalkyl or —$C_6H_5$ or —$CH_2C_6H_5$ (which may be substituted by halogen, methoxy, ethoxy or $C_1$ to $C_3$-alkyl groups) ;
n = 0 or 1 or 2 ;
m = 2 to 6, preferably 2 to 4.

2. The process as claimed in claim 1, wherein, in the supported catalyst, the chelate-forming organo-silicon compound is bonded, as the polyfunctional coupling agent, on the one hand to the support material and on the other hand alternately to the noble-metal compound and to a non-noble-metal compound from the sub-group 6 or 8 of the Periodic Table of the elements.

3. The process as claimed in claim 1 or 2, wherein the supported catalyst additionally contains, as promoters, non-noble-metal compounds from main groups 1 to 3 or sub-groups 4 to 6 or 8 of the Periodic Table of the elements.

4. The process as claimed in any one of claims 1-3, wherein the supported catalyst contains an inorganic oxidic support material or an activated charcoal support, whose residual active hydroxyl groups have been deactivated by esterification or etherification.

5. The process as claimed in any one of claims 1-4, wherein the supported catalyst contains a total of 0.01 to 50% by weight, preferably 0.1 to 20% by weight, of noble-metal compound, coupling agent and optionally non-noble-metal compound.

6. The process as claimed in any one of claims 1-5, wherein the supported catalyst is employed in a grain size of from 1 to 20 mm.

## Revendications

1. Procédé de préparation d'anhydrides monocarboxyliques de formule générale $(RCO)_2O$ par réaction entre un ester d'acide carboxylique ou un dialkyléther de formules générales RCOOR ou ROR, R désignant chaque fois le même radical alkyle possédant 1 à 4 atomes de carbone, et du monoxyde de carbone en phase gazeuse, en présence d'iode ou de brome ou de leurs composés jouant le rôle de promoteurs de la réaction ainsi qu'en présence d'un catalyseur supporté à des températures comprises entre 130 et 400°C et des pressions comprises entre 1 et 150 bar, un composé organosilicique à groupements alcoxy ou halogènes ainsi qu'à groupements organoazotés, organophosphorés, organoarséniés, organosulfuriques ou mercapto dans le catalyseur supporté étant lié en tant qu'adhésif à plusieurs fonctions d'une part à un matériau support et d'autre part à un composé de métal noble du groupe VIII du système périodique des éléments, caractérisé en ce qu'un composé organosilicique chélateur de formule générale

a) 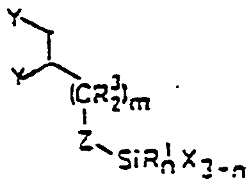 ou b) 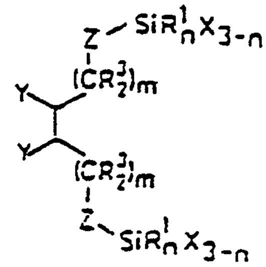

est utilisé comme adhésif à plusieurs fonctions, auquel cas

X = —Cl, —Br ou —$OR^2$ ;
Y = —$NR^4_2$, un radical aryle azoté, —$PR^4_2$, $AsR^4_2$, —$SR^4$ ou —SH ;
Z = 0, un arylène, un phénylène (éventuellement ortho-, méta- ou parasubstitué)
$R^1$ = un alkyle en $C_1$ à $C_5$ ;
$R^2$ = un alkyle en $C_1$ à $C_5$ ou —$C_6H_5$ ;
$R^3$ = —H, un alkyle en $C_1$ à $C_3$ ;
$R^4$ = un alkyle en $C_1$ à $C_6$, un cycloalkyle en $C_5$ ou $C_6$ ou —$C_6H_5$ ou —$CH_2C_6H_5$, substitués le cas échéant par des groupements halogènes, méthoxy, éthoxy ou alkyles en $C_1$ à $C_3$ ;
n = 0 ou 1 ou 2 ;
m = 2 à 6, de préférence 2 à 4.

2. Procédé selon la revendication 1, caractérisé en ce que dans le catalyseur supporté le composé organosilicique chélateur jouant le rôle d'adhésif à plusieurs fonctions est fixé d'une part au matériau support et d'autre part alternativement au composé de métal noble et à un composé de métal non noble du sous-groupe 6 ou 8 du système périodique des éléments.

3. Procédé selon la revendication 1 ou 2, caractérisé en ce que le catalyseur supporté contient en outre des composés de métaux non nobles des groupes 1 à 3 ou des sous-groupes 4 à 6 ou 8 du système périodique des éléments, jouant le rôle de promoteurs.

4. Procédé selon l'une quelconque des revendications 1 à 3, caractérisé en ce que le catalyseur supporté contient un matériau support oxydé inorganique ou un support en charbon actif dont les groupements hydroxyle actifs résiduels ont été désactivés par estérification ou éthérification.

5. Procédé selon l'une quelconque des revendications 1 à 4, caractérisé en ce que le catalyseur supporté contient au total 0,01 à 50% en poids, de préférence entre 0,1 et 20% en poids, de composé de métal noble, d'adhésif et le cas échéant de composé de métal non noble.

6. Procédé selon l'une quelconque des revendications 1 à 5, caractérisé en ce que le catalyseur supporté est utilisé à une granulométrie comprise entre 1 et 20 mm.